# EUROPEAN PATENT APPLICATION

(11) **EP 4 129 273 A1**
(43) Date of publication of application: **08.02.2023**
(21) Application number: 21780504.3
(22) Date of filing: 05.04.2021
(51) Int. Cl.: A61K 9/50, A61K 35/17, C12N 5/00

(54) **CELL CAPSULATING LAYER, CAPSULATED CELLS, CELL CAPSULATING COMPOSITION AND PREPARATION METHOD THEREFOR**

(30) Priority: 03.04.2020 KR 20200040814; 01.04.2021 KR 20210042722
(71) Applicant: Seoul National University R & DB Foundation, Seoul 08826 (KR)
(72) Inventor: HWANG, Nathaniel Suk-Yeon, Seoul 08826 (KR); KIM, Byung-Gee, Seoul 08826 (KR); KIM, Minji, Seoul 08826 (KR)
(74) Representative: Schiweck Weinzierl Koch Patentanwälte Partnerschaft mbB
(86) International application number: PCT/KR2021/004203
(87) International publication number: WO 2021/201660

(57) **Abstract**

The present invention relates to a method of co-incubating multiple cells. According to the present invention, an interaction between the multiple cells is facilitated without causing cytotoxicity, and a single cell can be separated without damage to the cells. Therefore, the present invention can be applied to a study of regeneration on various types of tissue cells.

## Description

### [TECHNICAL FIELD]

The present invention relates to a cell encapsulation layer that forms a hydrogel film on the surface of cells, a composition for cell encapsulation, and a method of preparing the composition for cell encapsulation.

This application claims the benefit of priorities based on Korean Patent Application No. 10-2020-0040814 filed on April 3, 2020, and Korean Patent Application No. 10-2021-0042722 filed on April 1, 2021, and all contents disclosed in those Korean patent applications are incorporated as a part of this specification.

### [BACKGROUND ART]

Cell therapeutic agents are drugs that transplant or inject cells such as a stem cell, an immune cell, and a beta cell into the body, and are attracting attention for treatment of severe diseases. Commercial interest in the cell therapeutic agents that use the cells, as pharmaceuticals, such as a CAR-T cell, which is the representative cell therapeutic agent for a blood cancer, the beta cell, which is the cell therapeutic agent for type 1 diabetes, and the stem cell therapeutic agent, is growing to apply these cells to a liver cirrhosis, an arthritis, and a cancer that are difficult to treat in the modern medical field.

Since the cells used as the therapeutic agents have a problem in that a survival rate thereof is greatly reduced due to physical stress and immune response when transplanted into the body, a technology for cell encapsulation has been studied in the fields of a tissue engineering and a regenerative medicine. Conventional platforms for cell delivery include a microbead technology using an alginic acid, a layered coating technology for the cells, and the like.

However, the microbead technology has a problem in that supply and exchange of materials outside the capsule, such as a nutrient and an oxygen, are limited due to a hydrogel too thick compared to a size of the cells, so that the cells are necrotic or their function is deteriorated. In addition, since the layered coating technology for the cells utilizes an attraction force between negatively and positively charged polymers, which results in generating a relatively weak electrostatic force in the bonding strength, the thickness of the coated layers is relatively thin so that the coated layers can be easily removed by a physical stress from the outside such as a pressure during the injection process and a blood flow, whereby there is limitation in stability, persistence, and durability.

Therefore, there has been required for a study on the cell encapsulation that does not affect the survival rate and proliferation of the cells after encapsulating the cells, by protecting the cells from the stress of the external environment to facilitate exchange of the substances simultaneously with increasing the survival rate.

### [DETAILED DESCRIPTION OF THE INVENTION]

### [TECHNICAL PROBLEM]

A purpose of the present invention is to provide a cell encapsulation layer.

Another purpose of the present invention is to provide a composition for cell encapsulation.

Still another purpose of the present invention is to provide an encapsulated cell.

Still yet another purpose of the present invention is to provide a method of preparing the composition for cell encapsulation.

### [TECHNICAL SOLUTION]

In order to solve the above problems, the present invention provides a cell encapsulation layer comprising a chitosan and a hyaluronic acid, wherein the chitosan and hyaluronic acid form cross-linking with each other.

According to an embodiment, the chitosan and the hyaluronic acid may form the cross-linking by an enzymatic reaction.

According to an embodiment, the chitosan and the hyaluronic acid may form the cross-linking by an aryloxy coupling.

According to an embodiment, the chitosan and the hyaluronic acid may be each independently functionalized with phenol, catechol or quinone, and specifically may be functionalized with, for example, the phenol.

According to an embodiment, a thickness of the cell encapsulation layer may be 50 nm to 500 nm, and specifically may be, for example, 100 nm to 200 nm.

According to an embodiment, a molecular weight of the chitosan may be 15 kDa to 310 kDa.

Further, a molecular weight of the hyaluronic acid may be 1.2 kDa to 8,000 kDa.

According to another embodiment of the present invention, there is provided a composition for cell encapsulation comprising a cell encapsulation layer and cells as described above.

According to an embodiment, the chitosan may be in direct contact with the cells.

According to an embodiment, the composition may comprise at least two cell encapsulation layers.

According to an embodiment, the cell encapsulation layer may include 1 to 5 chitosan layers and 1 to 5 hyaluronic acid layers.

According to another embodiment of the present invention, there is provided a method of preparing the composition for cell encapsulation as described above, the method comprising the steps of:
preparing a solution containing (i) a chitosan functionalized with a first moiety group and (ii) a hyaluronic acid functionalized with a second moiety group;
adding an enzyme to the solution to crosslink the polymers (i) and (ii); and
adding cells to the solution before, during or after addition of the enzyme.

According to an embodiment, the first moiety group and the second moiety group may each independently contain phenol, catechol, or quinone, and specifically may contain, for example, the phenol.

According to an embodiment, the cross-linking may include an aryloxy coupling.

According to an embodiment, the enzyme may include tyrosinase, and specifically may include, for example, tyrosinase derived from Streptomyces avermitilis (Streptomyces avermitilis-derived tyrosinase).

According to an embodiment, the polymer (i) in the solution may contain 0.01% to 5% of a chitosan modified with at least one aryl group.

In addition, the polymer (ii) in the solution may contain 0.01% to 5% of a hyaluronic acid modified with at least one aryl group.

According to another embodiment of the present invention, there is provided cells encapsulated with the cell encapsulation layer as described above.

Specific details on other embodiments of the present invention are shown in the detailed description of invention below.

### [ADVANTAGEOUS EFFECTS]

According to the present invention, it is possible to provide a composition for cell encapsulation that is stable and easy to exchange substances between materials outside a capsule coating layer and cells, while minimizing toxicity to the cells. In addition, since the composition of the present invention makes possible it to encapsulate a single cell or multiple cells in units of the single cell or the cell clusters and to encapsulate by coating an organ, it can be easily applied as a platform for a cell carrier such as stable cell transplantation and injection in the fields of a tissue engineering and a regenerative medicine, thereby achieving the technologies such as cell imaging and local site delivery.

### [BRIEF DESCRIPTION OF THE DRAWINGS]

FIG. 1 is a chemical formula showing a process of introducing a phenol moiety into a polymer.
FIG. 2 is structural comparison diagrams of the conventional mushroom-derived tyrosinase and actinomycete-derived tyrosinase.
FIG. 3 is a schematic diagram illustrating a process of cross-linking a polymer having a phenol moiety introduced thereto.
FIG. 4 shows the results of confirming that a monophenol moiety is introduced into a glycol chitosan and a hyaluronic acid polymer.
FIG. 5 is graphs showing a reaction rate between a polymer and tyrosinase.
FIG. 6 is a schematic diagram illustrating a process of cell encapsulation.
FIG. 7 is a diagram schematically illustrating encapsulation principle.
FIG. 8 is a schematic diagram and a photograph showing encapsulated cells according to the present invention.
FIG. 9 is photographs observing clustering phenomenon between the cells according to GC-T concentration.
FIG. 10 is a graph confirming cytotoxicity of actinomycetes-derived tyrosinase.
FIG. 11 is graphs showing the degree of cell encapsulation depending on concentration of an enzyme, reaction time, and concentration of a polymer.
FIG. 12 is graphs showing whether or not cell encapsulation is formed according to tyrosinase and electrostatic force.
FIG. 13 is a graph confirming whether or not a hydrogel nanofilm is formed by a quartz microscale method.
FIG. 14 is a graph showing zeta potential of encapsulated cells.
FIG. 15 is graphs showing FACS results of encapsulated cells.
FIG. 16 is photographs showing CLSM results of an encapsulated single cell.
FIG. 17 is photographs showing CLSM results of encapsulated β-cell spheroids.
FIG. 18 is photographs showing SEM results of encapsulated cells.
FIG. 19 is photographs showing TEM results of encapsulated cells.
FIG. 20 shows the results of confirming the degree of encapsulation of cells according to the laminated number of encapsulation layers.
FIG. 21 shows the results of confirming a survival rate of encapsulated cells.
FIG. 22 is the results of confirming persistence of cell encapsulation layer according to the presence or absence of a cross-linking of enzymes.
FIG. 23 is the results of confirming a response of encapsulated cells to glucose.
FIG. 24 is the results of confirming protease and physical stress resistance of encapsulated cells.
FIG. 25 is the results of confirming cytokine resistance of encapsulated cells.
FIG. 26 is a schematic diagram and the results of co-incubation of encapsulated cells and natural killer cells (NK cells) .

### [DETAILED DESCRIPTION OF THE EMBODIMENTS]

Since the present invention may apply various modifications and have various embodiments, specific embodiments are intended to be illustrated in the drawings and described in the specification in detail. However, these descriptions are not intended to limit the present invention to the specific embodiments, but should be understood to include all modifications, equivalents, or substitutions involved in the spirit and scope of the present invention. If it is considered that the detailed descriptions of related known technologies may obscure the gist of the present invention, those descriptions will be omitted.

Hereinafter, a cell encapsulation layer, a composition for cell encapsulation, and a method of preparing the composition for cell encapsulation according to an embodiment of the present invention will be described in more detail.

The present invention provides a composition for cell encapsulation having an appropriate thickness and a strong bond of a coating layer to facilitate exchange of substances inside and outside a capsule, so that problems of limiting an exchange of substances outside microbeads and degrading a stability in a coating method for laminating cells can be settled.

Specifically, the composition for cell encapsulation according to the present invention comprises a chitosan and a hyaluronic acid, wherein the chitosan and the hyaluronic acid form cross-linking with each other. A cell encapsulation layer includes encapsulated cells. The encapsulated cells may be encapsulated with one cell, or may be encapsulated with two or more cells.

According to an embodiment, the chitosan may contain a derivative thereof. Concretely, for example, the chitosan may contain glycol chitosan and have a molecular weight of, for example, 15 kDa to 310 kDa, and preferably, 75 kDa to 310 kDa.

Further, for example, a molecular weight of the hyaluronic acid may be 1.2 kDa to 8,000 kDa, and preferably, 40 kDa to 64 kDa.

The 'cross-linked bond' may be used synonymously with a 'bridge bond' or a 'cross-linking'. Also, it means a completely chemical bond such as a covalent bond or an ionic bond between a molecule and a molecule. For example, the chitosan and the hyaluronic acid in the present invention may form the covalent bond with each other, and may be crosslinked by an aryloxy coupling.

According to an embodiment, the chitosan and the hyaluronic acid may each independently be functionalized with phenol, catechol or quinone, and specifically, may be functionalized with, for example, phenol respectively, for example, mono-phenol.

As described above, the present invention provides the composition for cell encapsulation comprising a cell encapsulation layer and cells.

According to another embodiment of the present invention, there is provided cells encapsulated with the cell encapsulation layer as described above.

According to an embodiment, the cells to be encapsulated may be encapsulated with a single type of cells or one or more types of multiple cells without any limitation. Further, it is possible to encapsulate each of the cells or encapsulate a cell cluster at once, and it is also possible to encapsulate by coating an organ.

According to an embodiment, the chitosan contained in the cell encapsulation layer may be in direct contact with the cells.

According to an embodiment, the composition for cell encapsulation according to the present invention may form a film in which 1 to 10 layers, for example, 2 to 8 layers, preferably 3 to 7 layers, are laminated on the outside of the cells. Specifically, for example, the composition may comprise at least two cell encapsulation layers, and each independently comprise 1 to 5 layers, for example, 1 to 3 layers, of the chitosan and the hyaluronic acid.

Compared to the cell spherical encapsulation using the alginate beads of the prior art, the encapsulation layer in the form of the thin film of the multiple layers (Layer-by-layer, LBL) according to the present invention is formed of a very thin polymer layer to allow the cells to respond rapidly to detection of glucose and secretion of insulin. Specifically, the prior art has used the alginate beads having a size of 500 microns, which limits the rapid response of the cells to detect the glucose and secrete the insulin because the glucose must move several hundred microns into the alginate beads.

Electrostatic interaction between the polymers has been conventionally applied to form a thin-film coating of the multiple layers, but such manner has a disadvantage in that it requires an excessively long preparation time to achieve optimal condition of the cell encapsulation.

The present invention can solve the above disadvantage by inducing fast cross-linking using an enzyme.

According to another embodiment of the present invention, there is provided a method of preparing a composition for cell encapsulation, the method comprising the steps of:
preparing a solution containing (i) a chitosan functionalized with a first moiety group and (ii) a hyaluronic acid functionalized with a second moiety group;
adding an enzyme to the solution to crosslink the polymers (i) and (ii); and
adding cells to the solution before, during or after addition of the enzyme.

According to an embodiment, the first moiety group and the second moiety group may each independently contain phenol, catechol, or quinone, and may preferably contain phenol. The cross-linking between the polymers can be induced by introducing the first moiety group and the second moiety group into the chitosan and hyaluronic acid polymers, respectively. For example, a monophenol moiety may be introduced into glycol chitosan by treating the glycol chitosan with 4-hydroxyphenylacetic acid and 3-(4-hydroxyphenyl) propionic acid. Also, catechin, epigallocatechin gallate (EGCG), and other polyphenol compounds may be introduced.

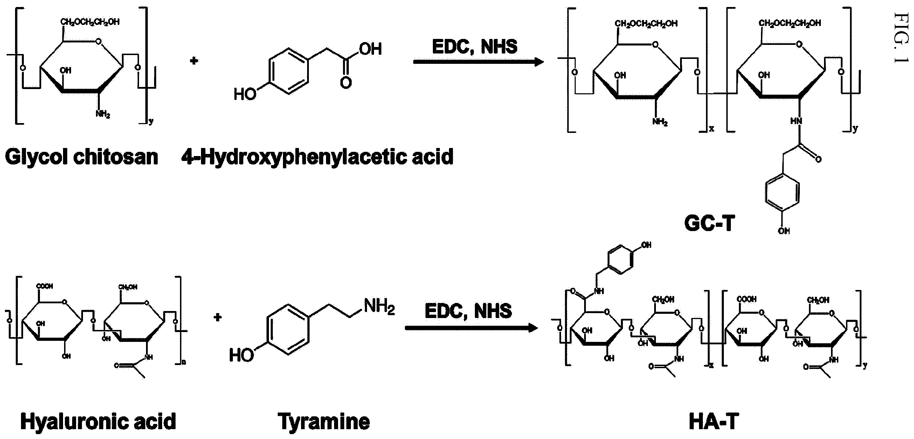

Further, the monophenol moiety may be introduced into the hyaluronic acid by treating the hyaluronic acid with at least one selected from the group consisting of tyramine and tyrosine. In addition, for example, the tyramine may be used in the form of a hydrochloride salt thereof. A process of introducing the phenol moiety according to an embodiment is shown in FIG. 1.

According to an embodiment, after the step of introducing the phenol into the first moiety group and the second moiety group, the chitosan and the hyaluronic acid can be crosslinked by enzymatic reaction which results from addition of one or more enzymes selected from the group consisting of monophenol oxidase, diphenol oxidase and laccase to the chitosan and the hyaluronic acid. Also, for example, the cross-linking may include an aryloxyl coupling.

According to an embodiment, the '(i) chitosan functionalized with the first moiety group' may contain a chitosan modified with at least one aryl group in an amount of 0.01% to 5%, for example, 0.1% to 1%, in the solution.

In addition, the ' (ii) hyaluronic acid functionalized with the second moiety group' may contain a hyaluronic acid modified with at least one aryl group in an amount of 0.01% to 5%, for example, 0.1% to 1%, in the solution.

According to an embodiment, the monophenol oxidase may include tyrosinase, and the diphenol oxidase may include catechol oxidase.

The tyrosinase is a metalloenzyme containing a copper and is present in various plants such as a mushroom, a potato and an apple, and animals. The tyrosinase acts as a cresolase that produces o-diphenol by oxidizing the mono-phenol, and acts as a catecholase that produces o-quinone by oxidizing the produced o-diphenol again.

The catechol oxidase has a function almost similar to that of the tyrosinase. However, the catechol oxidase can act as the catecholase that produces the o-quinone, but cannot act as the cresolase that oxidizes the mono-phenol, which is the other one of the actions of the tyrosinase.

The enzyme of the present invention may include tyrosinase, for example, tyrosinase derived from *Streptomes sp.* Specifically, for example, the enzyme of the present invention may include tyrosinase derived from *Streptomyces avermitilis (Streptomyces avermitilis*-derived tyrosinase). The tyrosinase derived from actinomycete of the *Streptomes sp.* has a higher catalytic efficiency for a tyrosine moiety bound to the polymer than that of a mushroom-derived tyrosinase, and thus shows a high conversion efficiency to dopa or quinone moiety. The converted moiety is cross-linked by a covalent bond or a coordination bond, and due to a dense cross-linking reaction, non-specific cross-linked bond is eliminated to shorten the cross-linking time and show high thermal and mechanical stability. Since the present invention uses only the cross-linked protein and the cross-linking tyrosine, it is possible to overcome the technical limitation of the prior art for the toxicity of monomeric chemicals and the limited accessibility of enzymes.

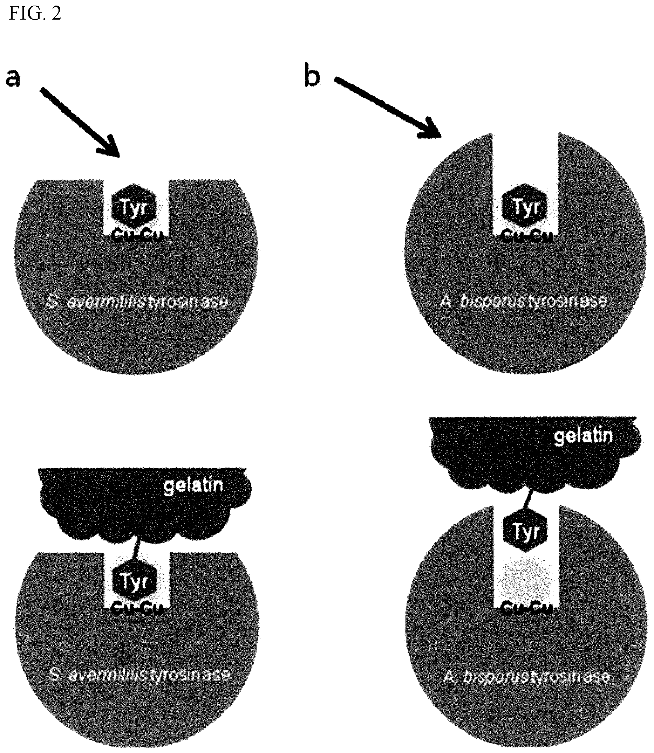

The actinomycete-derived tyrosinase is structurally different from the other species-derived tyrosinase as it has an open structure around the catalytic reaction and has very few steric hindrance factors. A structural comparison diagram of the mushroom-derived tyrosinase and the actinomycete-derived tyrosinase is shown in FIG. 2. FIG. 2a shows the actinomycete-derived tyrosinase, and exhibits that the steric hindrance around the entrance to which the tyrosinase accepts a substrate is small.

Contrary to this, FIG. 2b shows the mushroom-derived tyrosinase, and exhibits that accessibility to a tyrosine located in a protein of a large volume such as gelatin is reduced by a steric hindrance factor due to unnecessary peptides around the entrance. That is, the actinomycete-derived tyrosinase is advantageous for hydroxylation and oxidation reaction of the tyrosine with the polymer material, compared to the conventional mushroom-derived tyrosinase.

As a result, if reaction time of the conventional tyrosinase takes several hours, the tyrosinase derived from *Streptomyces avermitilis* can induce a quick and rapid reaction at several seconds. Therefore, in the step of adding the enzyme to crosslink the polymer, the reaction time may be 1 minute to 1 hour, for example, 1 minute to 30 minutes, or 3 minutes to 20 minutes, or 5 minutes to 15 minutes.

The phenol introduced into the chitosan and hyaluronic acid polymers can be oxidized by the enzyme as described above to form dopa or quinone, for example, o-quinone, and the dopa or quinone moiety can form a cross-linking by a coordination bond, that is, a covalent bond, to have a high stability due to a dense cross-linked structure.

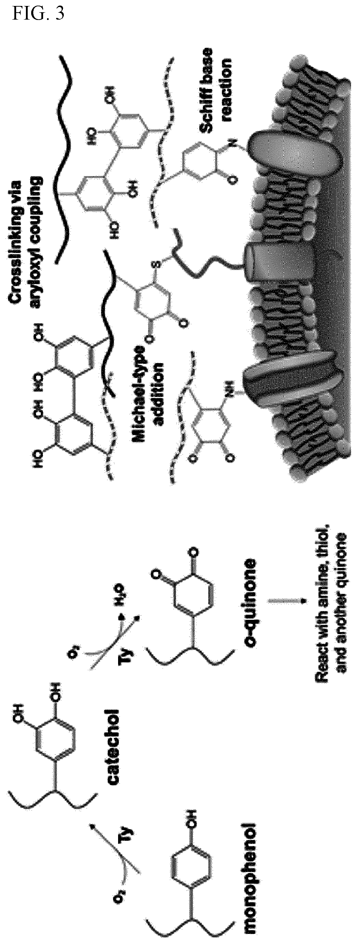

According to an embodiment of the present invention, the process of introducing the phenol group into the polymer and cross-linking it by oxidization is shown in FIG. 3. Compared with the conventional DOPA-modified macromolecules, the enzyme-based molecules according to the present invention are particularly efficient for oxidizing the phenol moiety, and can accelerate the cross-linking through a permanent covalent bond between catechol quinones, and amines, thiols and other phenol groups. In addition, since this enzyme itself, catechol, and catechol quinine-based substances do not have cytotoxicity or inflammation, a thin hydrogel can be formed on the cells using the tyramine-coupled chitosan and hyaluronic acid.

According to an embodiment, the step of adding the enzyme may include adding the tyrosinase of 0.01 to 0.5 µ/ml, for example 0.01 to 0.3 µ/ml, or 0.01 to 0.1 µ/ml, to the chitosan in a concentration of 0.01 to 1%, for example 0.05 to 0.5%, or 0.05 to 0.3% and the hyaluronic acid in a concentration of 0.01 to 1%, for example 0.05 to 0.5%, or 0.05 to 0.3%.

According to an embodiment, the step of adding the cells may include treating and immersing the cells on a membrane, and thus, for example, may seed the cells on the membrane to 1 × 10⁴ cells/ml to 1 × 10¹⁰ cells/ml. In addition, the membrane may have pores of 0.5 to 10 um, for example 1 to 8 µm, or 2 to 5 um. A type of the membrane may be selected, for example, one or more from the group consisting of polycarbonate (PC), cellulose, and polyvinylidene fluoride (PVDF), but is not particularly limited thereto.

According to an embodiment, the cell encapsulation layer according to the present invention may be formed in a type of the layered hydrogel nanofilm, and have a thickness of 50 to 500 nm, for example 100 to 300 nm, or 100 to 200 nm, wherein the thickness may be appropriately adjusted depending on the use, the application environment, and the like.

According to an embodiment, the cell encapsulation layer composition may be imaged by introducing a labeling material such as a fluorescent molecule or a light emitting molecule. In addition, drugs, growth factors, etc. may be introduced and delivered locally to an application target. For example, the cell encapsulation layer composition may be imaged by introducing a labeling material, for example, a chromogenic enzyme such as peroxidase, alkaline phosphatase, a radioactive isotope, a colloid, phycoerythrin (PE), fluorescein carboxylic acid (FCA), HRP, TAMRA, poly L-Lysine-fluorescein isothiocyanate (FITC), rhodamine-B isocyanate (RITC), rhodamine, cyanine, Texas Red, fluorescein, phycoerythrin, quantum dots, etc. Further, for example, an antibody epitope, a substrate, a cofactor, an inhibitor or an affinity ligand may be introduced.

As described above, since the composition for cell encapsulation according to the present invention forms a covalent bond between the polymers constituting the capsule by simultaneously applying an electrostatic attraction and an enzyme-mediated chemical reaction, it is not easily damaged by physical stimuli such as a pressure, a blood flow in the body, etc., upon injecting the encapsulated cells, thereby improving a stability and a persistence. In addition, regardless of the type of the cells, viable cells may be encapsulated in a single cell unit or in two or more types of multicellular units.

Further, according to the present invention, the cell encapsulation can be applied irrespective of a type of the cells, which makes possible improvement of the survival rate of cell clusters as well as biopharmaceuticals such as antibody proteins in the body. Since the encapsulation layer of the present invention can protect the cells from cytokine attack, it makes possible it to encapsulate by coating an organ, and can be applied as a platform for organ transplantation or a cell therapeutic agent, so that it is possible to prevent physical contact with immune cells in the body and to alleviate immune rejection.

Hereinafter, Examples of the present invention will be described in detail so that those of ordinary skill in the art can easily carry out the present invention. However, the present invention may be embodied in several different forms and is not limited to the Examples described herein.

### Example 1

### Introduction of a phenol moiety into a glycol chitosan and a hyaluronic acid

A monophenol moiety was introduced into each of glycol chitosan (GC) and hyaluronic acid (HA) polymers to synthesize chitosan-monophenol (GC-T) and hyaluronic acid-monophenol (HA-T). After dissolving 200 mg of the glycol chitosan in 10 ml of 0.1M pH 4.7 MES buffer, 160.82 mg of 4-hydroxyphenyl acetic acid (HPA) was dissolved in 10 ml of the MES buffer, and 202.64 mg of EDC and 114.76 mg of NHS was added to the solution and stirred for 5 minutes. Subsequently, the two solutions were mixed and reacted overnight at a room temperature. The solutions were then dialyzed against a distilled water for 72 hours and freeze-dried for at least 72 hours. After dissolving 200 mg of the hyaluronic acid in 20 ml of 0.1M pH 4.7 MES buffer, 197.452 mg of the EDC and 111.822 mg of the NHS were added and stirred for 5 minutes. Subsequently, 178.85 mg of tyramine hydrochloride was added and reacted at a room temperature overnight. The solution was then dialyzed against a distilled water for 72 hours and freeze-dried for at least 72 hours.

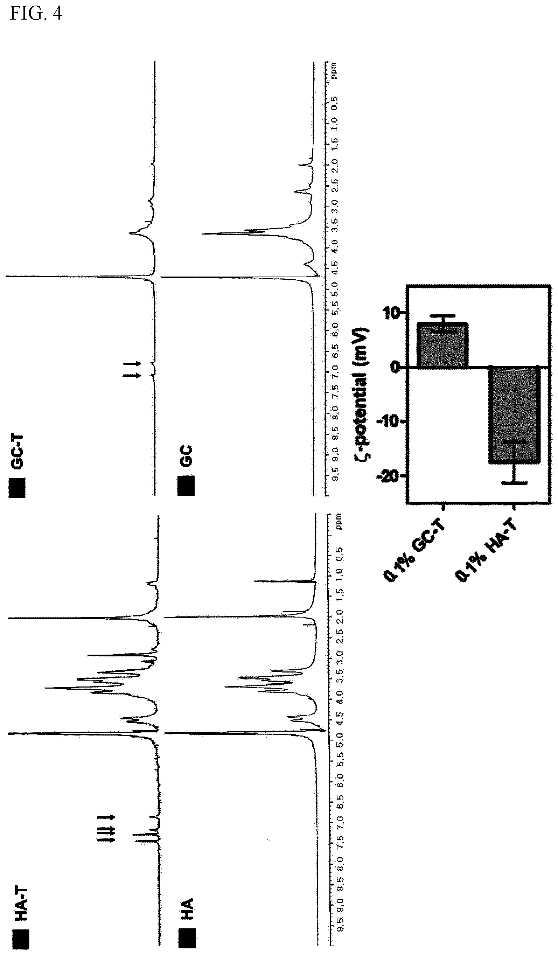

It was confirmed by a nuclear magnetic resonance spectroscopy (NMR Spectroscopy) that the monophenol moiety was introduced into the glycol chitosan and hyaluronic acid polymers, and the results were shown in FIG. 4.

Further, in order to synthesize a fluorescent polymer, 1 ml of 10 mg/ml RITC (Rhodamine B isothiocyanate/Sigma-Aldrich) and FA (fluoresceinamine isomer I/Sigma-Aldrich) solution dissolved in N, N-dimethylformamide (DMF) was added to a reaction solution of the GC-T and the HA-T, respectively, to synthesize GC-T-RITC and HA-T-FA.

### Reaction of tyrosinase with GC-T and HA-T

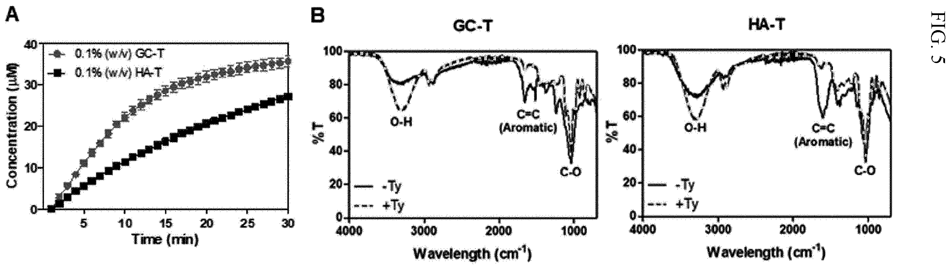

Tyrosinase was used as an actinomycete derived from *Streptomyces avermitilis* (SA). 2.5 uM of SA-derived tyrosinase (SA-Ty), 5 uM of CuSO₄ and a substrate were prepared in a total volume of 200 ul of 50 mM Tris-HCl buffer having pH 8.0. The substrate is 200 uM of either GC-T 1% (w/v), HA-T 1% (w/v) or L-tyrosine. Using a microplate reader (Infinite M200 PRO, TECAN, Switzerland), an absorbance was measured at 37°C at 475 nm (ε dopachrome = 3600 M⁻¹cm⁻¹) every 1 min for 30 min. The results were shown in FIG. 5, wherein the initial reaction rate of SA-Ty is defined as a slope of the concentration and the reaction time of the product. Enzyme activity is also expressed in units per ml (U/ml), in which one unit (U) is an amount of the SA-Ty catalyzing a L-tyrosine reaction at 1 pmol per minute.

### Cell encapsulation

The freeze-dried GC-T and HA-T were dissolved in PBS and 0.1% acetic acid at each 10 mg/ml. After complete dissolution, each solution was diluted 10-fold with the PBS to prepare it in a final concentration of 1 mg/ml, and filtered through a sterile 0.2 um membrane. The collected Jurkat cells were washed twice with the PBS and prepared at a density of 1×10⁷ per 1 ml of the PBS. 100 µl of the cell suspension was seeded on a 3.0 um polycarbonate membrane (Transwell^{®} 6.5 mm insert, 24-well plate, Corning^{®}). Cell encapsulation was performed by repeating the following method using GC-T solution for odd-numbered layers of layers 1, 3 and 5, and HA-T solution for even-numbered layers of layers 2, 4 and 6. After adding 600 µl of 1 mg/ml GC-T or HA-T solution and 0.05 U/ml SA-Ty to the 24-well plate, the cell-seeded inserts were immersed at a room temperature (RT) for 10 min.

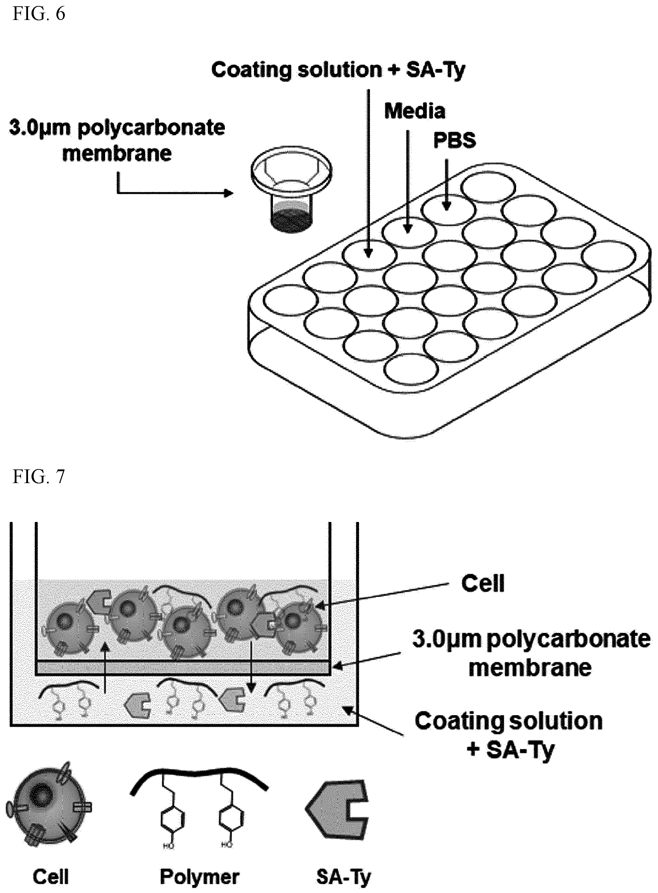

During the incubation, the plate was tapped every 2 min. After 10 min, the inserts in a shaking incubator were transferred to a well into which 500 µl of a medium (Roswell Park Memorial Institure 1640 (Gibco) containing 10% FBS (Corning)) was added for 30 seconds, and were transferred to a well into which 500 µl of the PBS was added for 1 min. Thereafter, the same method as described above was applied to the HA-T and GC-T to produce a final layer, and the cells were dispersed in a cell incubation medium (Roswell Park Memorial Institure 1640 (Gibco) containing 10% FBS (Corning)). A schematic diagram of the above process was shown in FIG. 6. Further, a specific encapsulation principle was schematically illustrated in FIG. 7.

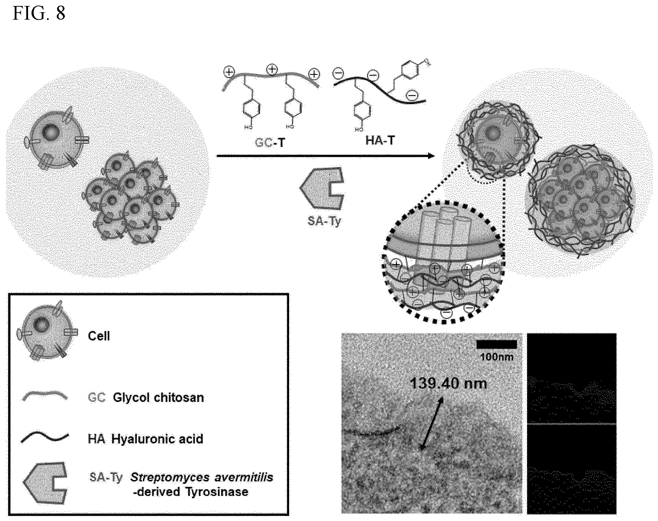

An appearance of the encapsulated cells prepared by the above method was shown in FIG. 8. FIG. 8 is a view of the encapsulated cells in which a hydrogel nanofilm is laminated in 6 layers. A visualization photograph of a red fluorescence (RITC)-labeled glycol chitosan layer and a green fluorescence (FA)-labeled hyaluronic acid layer can be confirmed from FIG.8. In addition, it is confirmed that a thickness of the hydrogel nanofilm laminated on the outside of the cells was 139.4 nm.

### Confirmation of cell clumping phenomenon depending on GC-T concentration

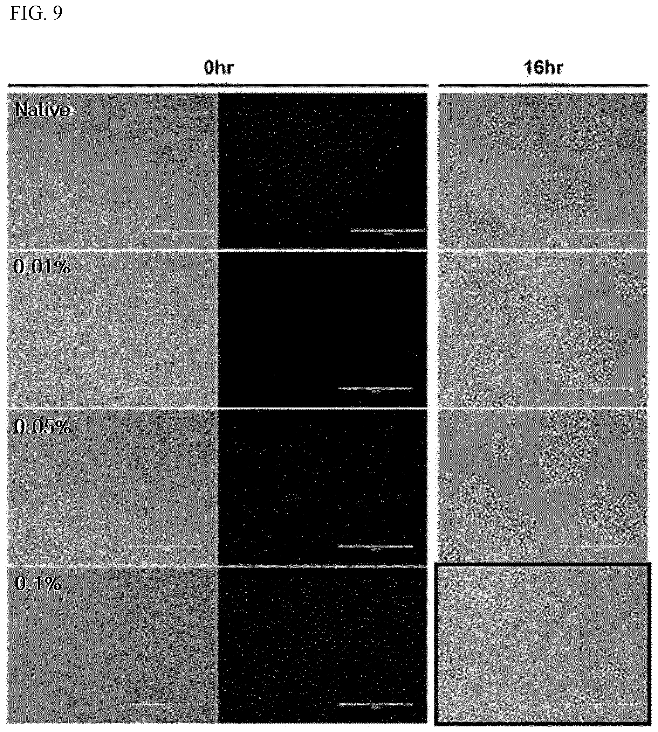

By varying a concentration of the glycol chitosan-monophenol (GC-T), the Jurkat cells having cell clumping property were encapsulated, and the results were shown in FIG. 9. When the cells were observed after 16 hours, it was confirmed that the cell clumping phenomenon was shown at a concentration of 0.05% or less, whereas a repulsion phenomenon between the cells occurred at a concentration of 0.1% or more. This is a phenomenon caused due to a charge of the glycol chitosan-monophenol (GC-T) encapsulated on the cell surface.

### Experimental Example 1: Confirmation of cytotoxicity of actinomycete-derived tyrosinase

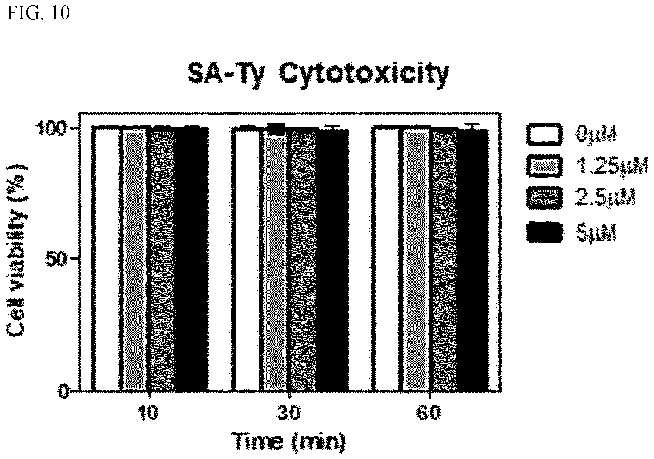

After *Streptomyces avermitilis* (SA)-derived tyrosinase (SA-Ty) was incubated by mixing it in media at a concentration of 0 to 5 µM for 10, 30, and 60 minutes, respectively, a survival rate of the cells was observed by a Live/Dead assay. The results were shown in FIG. 10 that indicates the survival rate of 97% or more among the overall cells. Therefore, it was confirmed that there was no toxicity when the cells were treated at a maximum of 5 µM for 60 minutes.

### Experimental Example 2: Optimization of cell encapsulation condition

In order to optimize coating of a cell encapsulation hydrogel film, a relationship between a SA-Ty concentration, a reaction time and a polymer concentration was analyzed. The degree of coating was checked by measuring fluorescence intensity of GC-T-RITC and HA-T-FA. MIN6 β cells seeded in a 96-well tissue incubation plate were incubated with 0.05 U/ml of SA-Ty in 0.1% of GC-T-RITC solution or 0.1% of HA-T-FA solution for 10 minutes. After washing with PBS, fluorescence intensity was measured at two different areas; among the measurement areas, one is λex = 543 nm/λem = 580 nm (RITC), and the other is λex = 495 nm/λem = 525 nm (FA). For Native (N), the MIN6 β cells, which were subjected to the same procedure using the PBS instead of the coating solution, were used.

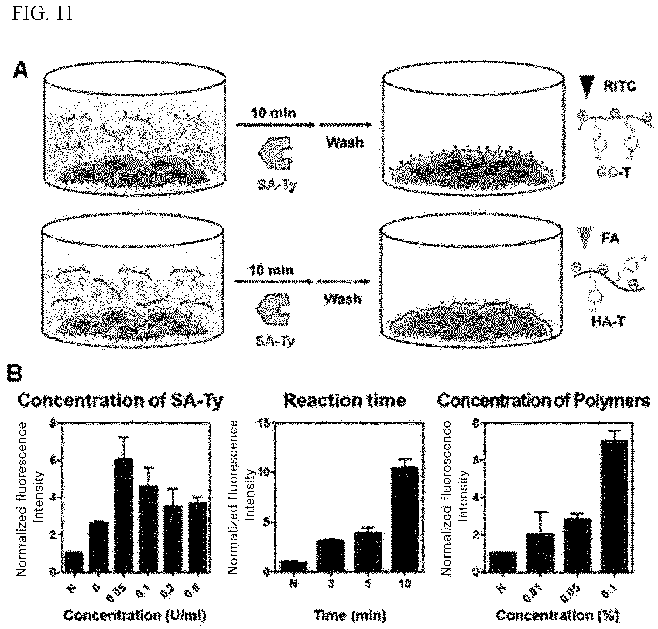

The results were shown in FIG. 11.

The highest RITC intensity was shown at a concentration of 0.05 U/ml of the SA-Ty, a reaction time of 10 minutes, and a concentration of 0.1% of the chitosan polymer. When the SA-Ty concentration was 0.05 U/ml and the chitosan polymer concentration was 0.1%, a coating level of the GC-T-RITC increased with longer the reaction time.

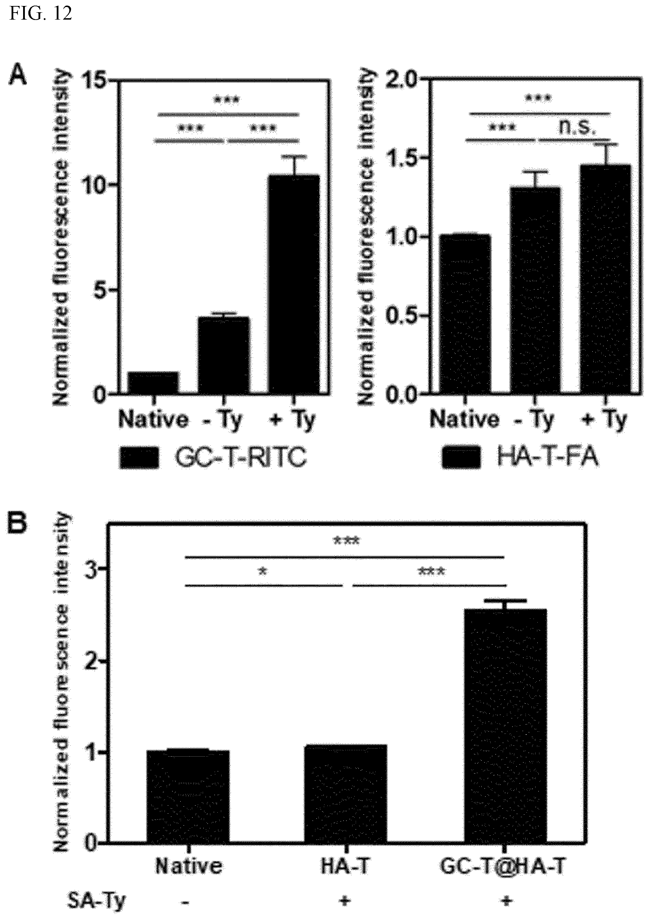

Further, a coating effect according to the presence of the SA-Ty in the GC-T-RITC and HA-T-FA solutions was investigated, respectively. In addition, an influence of the coating order was analyzed by comparing the cells coated with the single HA-T film to the cells coated with the HA-T film on the cells first coated with the GC-T film. The results were shown in FIG. 12. According to the presence of the SA-Ty, positively charged GC-T-RITC showed a difference of 2.9 times, whereas negatively charged HA-T-FA showed little difference in the degree of coating. In addition, the FA intensity was increased by 2.4 times when the HA-T film was coated on the GC-T film-coated cells.

### Experimental Example 3: Confirmation of cell encapsulation

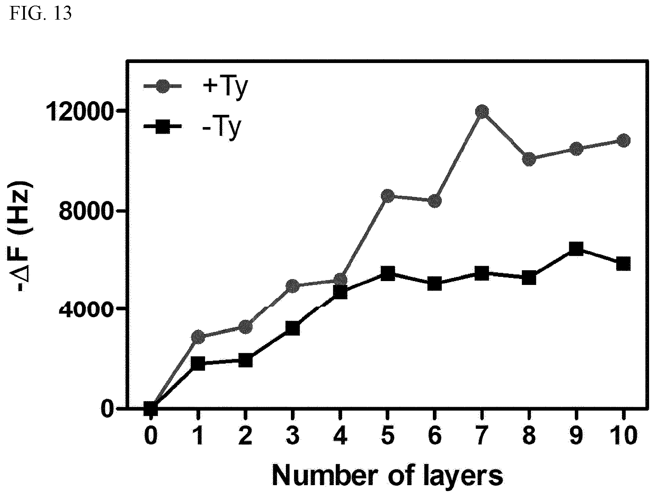

A Quartz Crystal microbalance method was used to confirm whether a hydrogel nanofilm was formed. A GC-T/HA-T layer was deposited using a Cr/Au (chromium/gold) crystal (5 MHz, 1 inch in a diameter, AT-cut, plane-plane). Prior to the deposition, the crystal was treated with a piranha solution (H₂SO₄:H₂O₂ = 3:1) for 5 min and an oxygen plasma for 5 min to clean the surface thereof and set a negative charge. Next, the crystal was immersed in 0.1% of the GC-T solution with 0.05 U/ml of the SA-Ty. After 10 min, the electrode was washed twice with PBS for 1 min. To remove the remaining PBS, the crystal was dried with a blower. A multilayered thin film (layer-by-layer (LBL)) deposition was performed with 0.1% of the HA-T solution until a total of five double layers were laminated. The crystal on which the hydrogel film was deposited was analyzed for each layer by the quartz crystal icrobalance (QCM, QCM200, Stanford Research Systems, USA) method, and the results were shown in FIG. 13.

Further, encapsulation of the cells was demonstrated.

### Zeta Potential

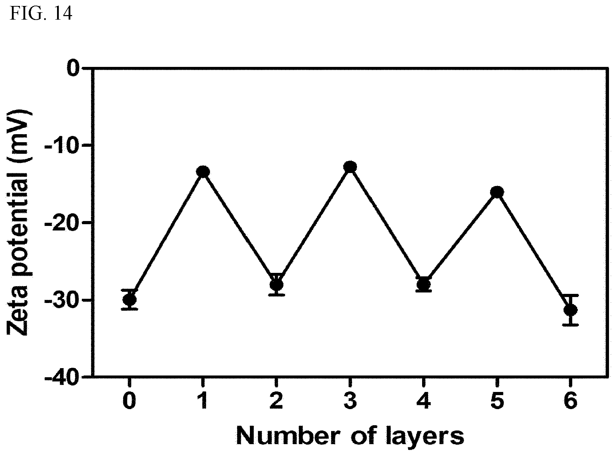

The Cells were fixed with 4% paraformaldehyde (PFA) for 10 minutes, and prepared in a density of 1 × 10⁶ cells per 1 ml of the PBS. Zeta potentials of native cells or encapsulated cells were measured with Nano ZS (Malvern Instruments, Germany) and were shown in FIG. 14.

### Fluorescence Activated Cell Sorter (FACS)

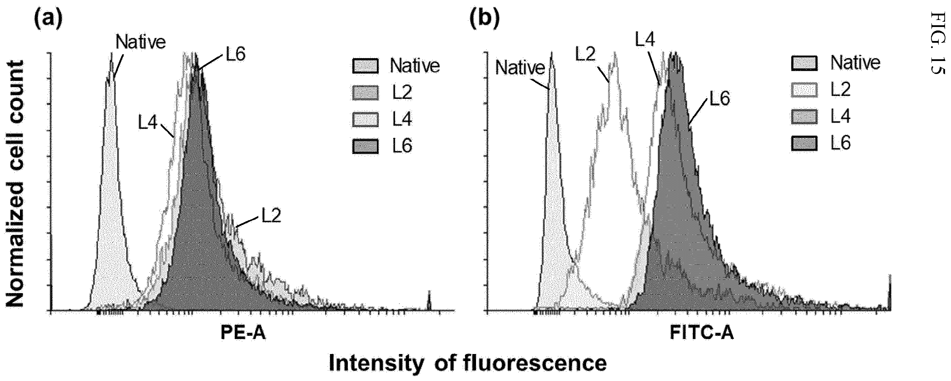

The cells were encapsulated with the GC-T-RITC and the HA-T-FA instead of the GC-T and the HA-T. After fixation, 1×10⁶ cells per 500 ul of the PBS were prepared. Fluorescence of the RITC and the FA was measured by a flow cytometry (FACS Aria II, BD Biosciences, USA) using a laser with wavelengths of 488 nm and 633 nm. The monolayered Cells encapsulated with the GC-T-RITC and the HA-T-FA were used as positive controls for gating, and the results were shown in FIG. 15.

### Confocal Laser Scanning Microscope (CLSM)

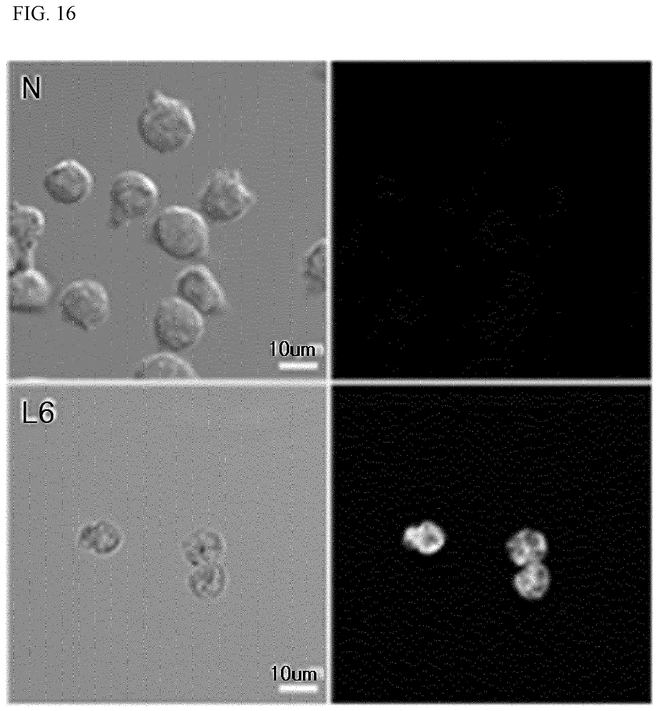

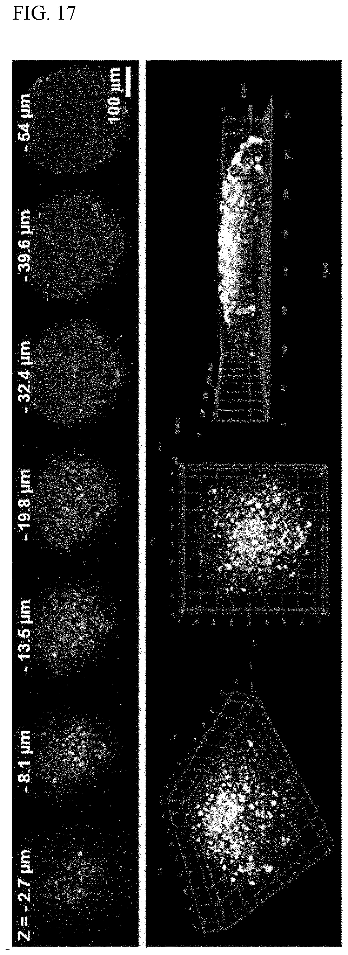

The Single cell or the β-cell spheroids (pancreatic cells) were encapsulated with the GC-T-RITC and the HA-T-FA, respectively, and then fixed with 4% PFA at a room temperature (RT) for 15 min. The spheroids in the form of an ellipsoid revolution were placed in a 20 mm confocal dish. They were imaged through a confocal microscope (LSM 780, Carl Zeiss, Germany). The results for the single cell were shown in FIG. 16, and the results for the β-cell spheroids were shown in FIG. 17. Since fluorescence appeared mainly on the outer surface of the encapsulated spheroids, it was confirmed that the hydrogel nanofilm was uniformly covered on the surface of macromolecules.

### Scanning Electron Microscope (SEM)

SEM (JSM-6701F, JEOL) images were analyzed to observe the surface of the encapsulated β-cell spheroids.

For preparation of samples for the SEM analysis, the spheroids were fixed with 2.5% glutaraldehyde solution for 4 hours, and dehydrated with ethanol. Then, they were dried through hexamethyldisilazane, and observed by carrying out platinum coating for 120 seconds.

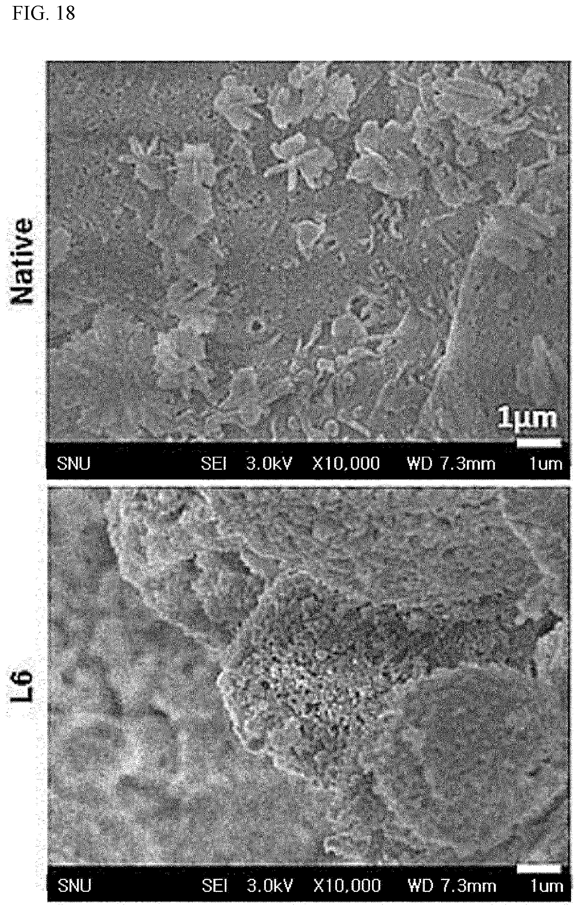

The results were shown in FIG. 18. When the GC-T is uniformly coated on the Jurkat cells, the cells repel each other and are coated with a uniform cover of the polymer. It was confirmed that the cross-linked polymer was evenly laminated on the surface of the encapsulated spheroids. The surface of the encapsulation layers formed a mesh structure.

On the contrary, the native spheroids had a smooth surface that is not coated with the polymer.

### Transmission Electron Microscope (TEM)

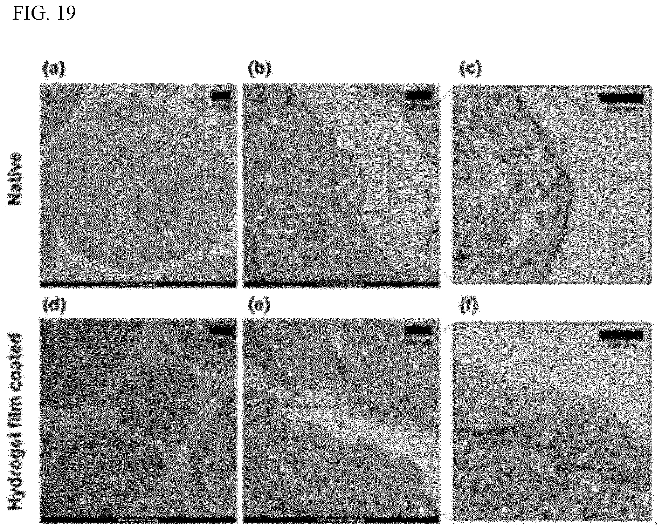

TEM (Tales L120C, 120kV, FEI, Czech) images were analyzed to confirm whether the hydrogel is formed in a thickness of nanometer (nm) on the cell surface. For preparation of TEM samples, the native cells and the encapsulated cells were fixed with a Karnovsky's fixing solution. The cells were treated with 1% osmium tetroxide in a cacodylate buffer for 1 hour, and then treated with 0.5% uranyl acetate at 4°C for one day. After dehydration with ethanol, the samples were embedded in a Spurr resin. The specimens were cut using an ultramicrotome (EM UC7, Leica, Germany), and the analysis results were shown in FIG. 19.

### Fluorescence Microscope

Fluorescence images were observed to confirm the degree of cell encapsulation for each lamination number of the encapsulated β-cell spheroids.

When the β-cell spheroids were encapsulated with the GC-T-RITC, the HA-T-FA, it was confirmed by imaging with a fluorescence microscope (EVOS^{®} Cell Imaging Systems, Thermo Fisher Scientific) that the fluorescence intensity increased as the number of laminations increased.

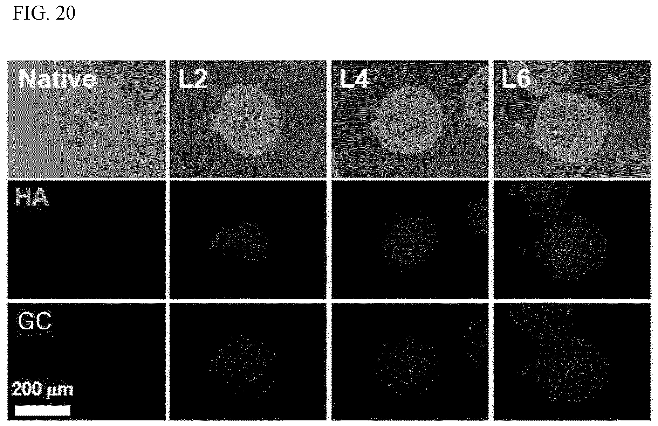

The results were shown in FIG. 20.

### Experimental Example 4: Confirmation of survival rate of encapsulated cells

In order to confirm a survival rate of the encapsulated cells, the cells were stained and analyzed with Live/Dead^{®} Viability/Cytotoxicity Kit containing calcein-AM and ethidium homodimer-1 (EthD-1).

The calcein-AM can be delivered to neighboring cells only through cell tunnels formed by direct contact between the cells. The calcein-AM, which has passed through the cell membrane, loses membrane permeability to stay in the cells as a bond of acetoxymethyl ester is broken by esterase in the cells and separated to calcein. Since an amount of green fluorescence measured with a microscope, a fluorometer, a flow cytometry, etc. is determined by the membrane permeability of viable cells and activity of the esterase, it reflects activity of the cells as it is.

The EthD-1 only enters dead cells having the cell membrane damaged, and emits red fluorescence by a bond to nucleic acids.

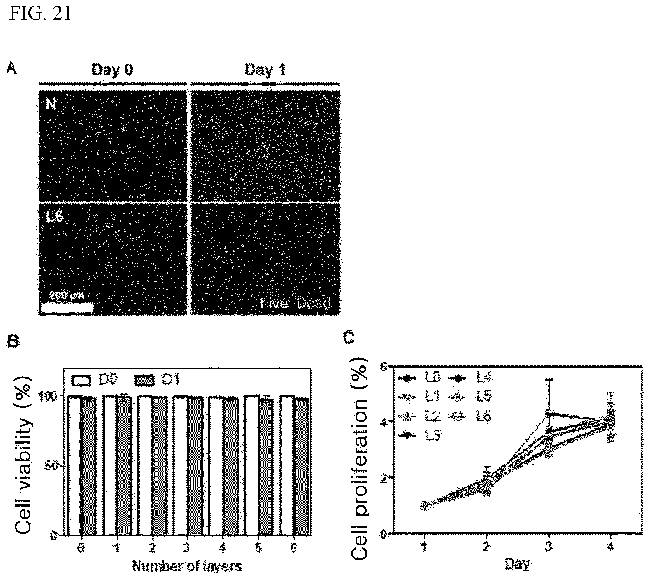

After the stained cells were imaged with a fluorescence microscope (EVOS^{®} Cell Imaging Systems, Thermo Fisher Scientific), the cells were counted in four separate fields to calculate a percentage of the viable cells. For proliferation, cell metabolism was measured for 3 or 4 days at intervals of 24 hours using an alamarBlue^{®} reagent. The measured fluorescence of the reagent was normalized to the value of the first day. The results were shown in FIG. 21. FIG. 21A is a photograph visualizing a survival rate of the unencapsulated Jurkat cells and the Jurkat cells encapsulated in 6 layers according to Example 1. FIG. 21B is a graph showing a survival rate of the cells according to the number of encapsulation layers of the hydrogel nanofilm. FIG. 21C is a graph showing a proliferation rate of the cells according to the number of encapsulation layers and time.

### Experimental Example 5: Confirmation of persistence of encapsulation layers

In order to confirm persistence of the cell encapsulation layers according to the presence or absence of crosslinking of an enzyme, fluorescence intensity of the encapsulation layers over time was checked.

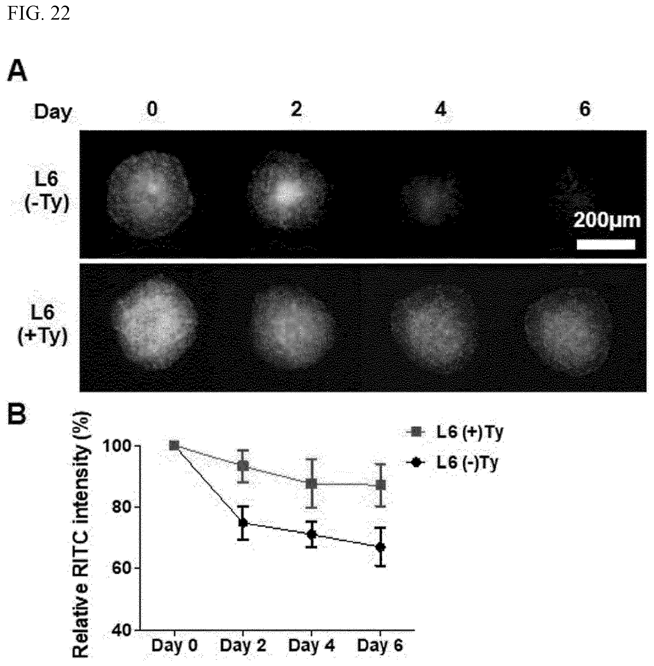

The β-cell spheroids were encapsulated using the GC-T-RITC and the HA-T-FA, and the persistence of the encapsulation layers with or without use (-Ty or +Ty) of the enzyme during encapsulation was imaged by a fluorescence microscopy (EVOS^{®} Cell Imaging Systems, Thermo Fisher Scientific). The results were shown in FIG. 22A. It was confirmed that when the enzyme was used (+Ty), the fluorescence was maintained until 6 days after the incubation of the cells outside the body.

Further, the cell layers in a 2D state were encapsulated using the GC-T-RITC and the HA-T, and likewise, the persistence of the encapsulation layers with or without use of the enzyme was measured every 24 hours with excitation at 543 nm and emission at 580 nm, using a microplate reader (Infinite M200 PRO, TECAN, Switzerland). The results were shown in FIG. 22B. It was confirmed that the encapsulated cells were inhibited from dissociation even at 48 hours.

### Experimental Example 6: Confirmation of response of encapsulated cells to glucose

Insulin ELISA (enzyme-linked immunosorbent assay) was performed to check an insulin secretory ability and a relative gene expression according to the number of laminations of the encapsulated cells. In the encapsulation layers of the present invention, low molecules such as glucose, amino acid, and insulin can freely diffuse into and out of the cells. The L6 encapsulation layers show a low diffusion rate for both the FITC-dextran of 20 kDa and 70 kDa, but does not interfere with the insulin secretion at 5.8 kDa.

An amount of the insulin secretion was measured by the ELISA of β-cell spheroids encapsulated using the GC-T and the HA-T after 1 day and 7 days, respectively.

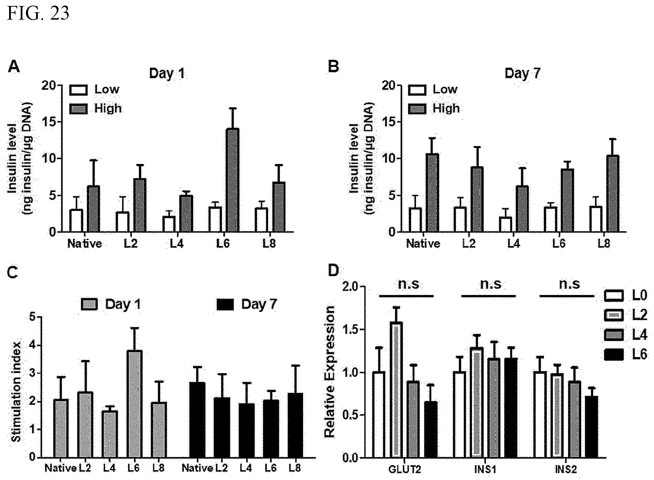

The results of checking insulin levels in the hypoglycemic and hyperglycemic solutions were shown in FIG. 23A and 23B.

Further, in order to analyze an amount of the insulin secretion, the encapsulated β-cell spheroid samples were incubated in 3.3 mM low-concentration glucose (D-Glucose) and 20 mM high-concentration glucose solutions for 2 hours, respectively, to obtain a supernatant thereof. Thereafter, a concentration of the insulin was analyzed using a mouse insulin ELISA (80INSMS-E01, ALPCO) kit.

Glucose sensitivity was investigated from a value obtained by dividing an amount of the insulin secretion in the high-concentration glucose solution by an amount of the insulin secretion in the low-concentration glucose solution, and the result were shown in FIG. 23C.

Further, a polymerase chain reaction was performed to compare expression levels of GLUT-2, INS-1, and INS-2 as glucose and insulin-related genes. The spheroid samples were transferred to a tube containing 500 ul of a trizol solution, and 100 µl of a chloroform solution was added thereto, followed by centrifugation at the conditions of 21,055 xg, 20 minutes and 4°C. After obtaining a transparent aqueous layer, 250 µl of isopropanol was added and centrifuged at the conditions of 21,055 xg, 20 minutes and 4°C. After removing a supernatant, the samples were washed with 500 ul of 75% ethanol, and the precipitated samples were dissolved in a distilled water. After denaturation at 60°C for 10 minutes, cDNA was prepared using EZ066M (Enzynomics, Korea) kit.

The expression levels of GLUT-2, INS-1 and INS-2 genes were analyzed using StepOnePlus^{™} Real-Time PCR system (Applied Biosystems) equipment and SYBR Green PCR Mastermix.

As a result, a graph showing changes in every number of laminations of the β-cell-related genes GLUT-2, INS-1 and INS-2 involved in the insulin synthesis and secretion was shown in FIG. 23D.

### Experimental Example 7: Confirmation of protease and physical stress resistance of encapsulated cells

Depending on the presence or absence of encapsulation of the β-cell spheroids, they were incubated in a solution of 0.05% Trypsin as the protease for 30 minutes, respectively, followed by light pipetting to observe the degree of degradation over time.

Further, depending on the presence or absence of encapsulation of the β-cell spheroids, they were incubated in a solution of a 10 mg/ml collagenase type 2 as the protease up to 18 hours in maximum to observe the degree of degradation.

In addition, the natural spheroid group and the encapsulated β-cell spheroid group were centrifuged at the conditions of 80 xg and 1073 xg for 5 minutes, respectively, to check the degree of resistance to physical stress (centrifugation) depending on the presence or absence of encapsulation of the β-cell spheroids.

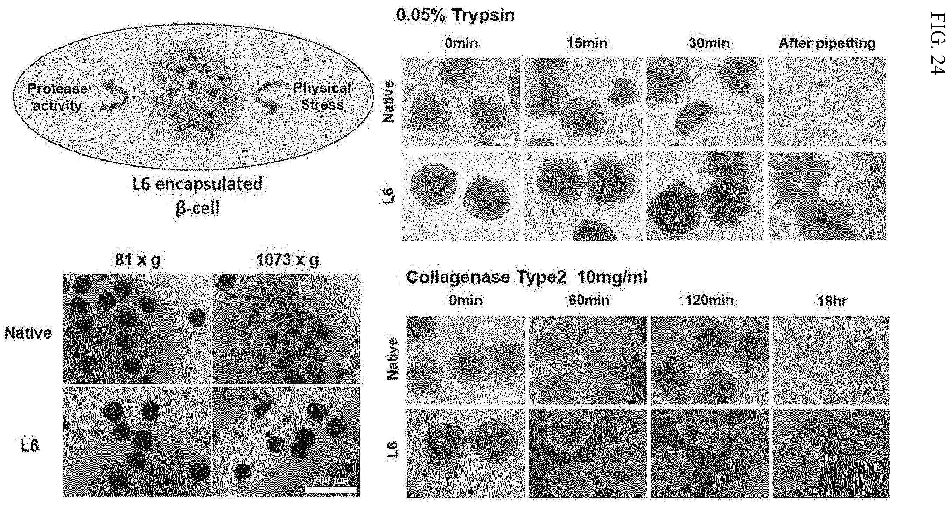

The results were shown in FIG. 24.

The natural spheroid group treated with the trypsin or the collagenase type 2 was collapsed and dissociated easily by light pipetting, whereas the encapsulated spheroid group showed little separation of the cells and maintained structure thereof.

Also, with respect to the physical stress, both the native spheroid group and the encapsulated spheroid group maintained their spherical shapes at a low centrifugal speed, but the native spheroid group was collapsed at a high centrifugal speed. Contrary to this, it was confirmed that the L6 encapsulated spheroid group had a persistence due to withstanding a high pressure.

### Experimental Example 8: Confirmation of resistance of encapsulated cells to cytokine

Resistance of the encapsulated β-cell spheroids to a cytokine was checked.

TNF-α, an inflammation-inducing cytokine, which induces apoptosis of β-cells, was analyzed through PCR to determine whether the encapsulation layers block access of the TNF-α. After 48 hours under an incubating condition of adding the TNF-α in a concentration of 10 nM, the expression level of apoptosis-related genes was compared in the natural spheroid group and the encapsulated spheroid group through the PCR. The PCR process was as described above, and p53 and Caspase-3 were selected as the genes.

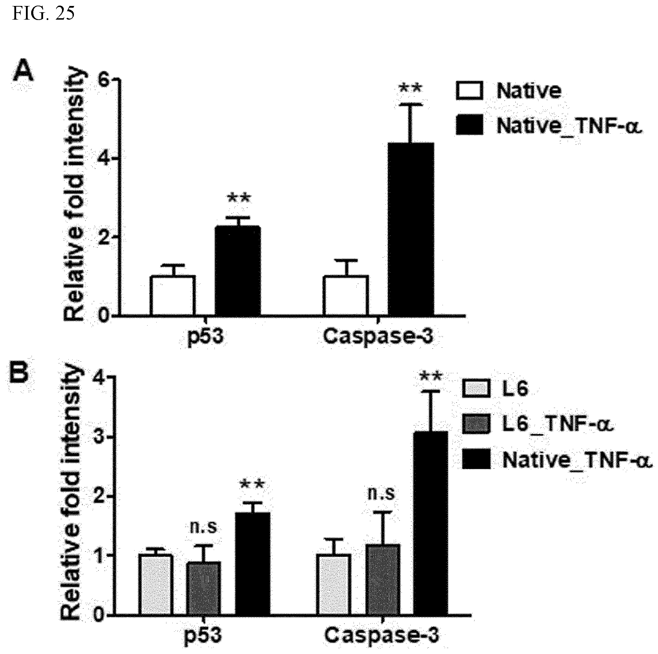

The results were shown in FIG. 25. It was confirmed that the encapsulated spheroid group did not respond to the TNF-α treatment as it protected an attack of the cytokine. These results show that the cytokines having a molecular weight close to 20 kDa cannot diffuse through the L6 encapsulated cells having the six-layered hydrogel nanofilm.

Contrary to this, the natural spheroid group indicated that the cell genes such as the p53 and the Caspase-3 involved in the cell cycle and the apoptosis were significantly increased.

### Experimental Example 9: Confirmation of blocking of encapsulated cells against immune cells

In order to check whether the encapsulated cells block immune cells, the encapsulated β-cell spheroids and the natural killer cells (NK92c cells) were co-incubated. By cell-cell contact, it was checked whether the encapsulation layers block contact of the immune cells through the co-incubation with the natural killer cells that attack the β-cells.

The β-cell spheroid samples and the natural killer cells (NK-92 cells) were fluorescently stained using CellTracker TM Green (Invitrogen) and CellTrace Far-red (Invitrogen), respectively, and were incubated and imaged using Chamlide TC incubator system (Live-cell Instrument, Korea).

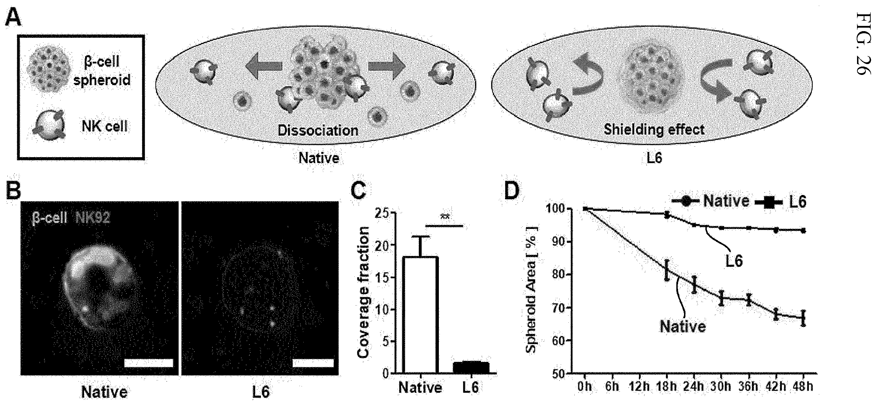

The results were shown in FIG. 26. It was confirmed that the encapsulated β-cell spheroids had little contact with the natural killer cells and a size of the spheroids was also maintained. In contrast, it was confirmed that the natural spheroids had high contact with the natural killer cells and an area of the spheroids was rapidly reduced.

As can be seen from the above results, a nanometer-thick hydrogel nanofilm can be formed on the outside of the cells without toxicity to the cells, which leads to improve stability by forming a covalent bond between the polymers constituting the nanofilm. In addition, since an appropriate thickness can be easily formed to the hydrogel nanofilm, the exchange of substances between the cells and the external environment is facilitated, thereby protecting the cells to maintain their function.

The above descriptions are merely illustrative of the technical idea of the present invention, and various modifications and variations are possible for those of ordinary skill in the art to which the present invention pertains without departing from the essential characteristics of the present invention. Also, the Examples described in the present invention are not intended to limit the technical spirit of the present invention, but to illustrate the invention, and thus the scope of the technical spirit of the present invention is not limited by these Examples. The protection scope of the present invention should be construed by the following claims, and all technical ideas within the scope equivalent thereto should be understood as being included in the scope of the present invention.

## Claims

1. A cell encapsulation layer comprising a chitosan and a hyaluronic acid, wherein the chitosan and hyaluronic acid form cross-linking with each other.

2. The cell encapsulation layer according to claim 1,
wherein the chitosan and the hyaluronic acid form the cross-linking by an enzymatic reaction.

3. The cell encapsulation layer according to claim 1,
wherein the chitosan and the hyaluronic acid form the cross-linking by an aryloxy coupling.

4. The cell encapsulation layer according to claim 1,
wherein the chitosan and the hyaluronic acid are each independently functionalized with phenol, catechol, or quinone.

5. The cell encapsulation layer according to claim 1,
wherein the chitosan and the hyaluronic acid are functionalized with the phenol.

6. The cell encapsulation layer according to claim 1,
wherein a thickness of the cell encapsulation layer is 50 nm to 500 nm.

7. The cell encapsulation layer according to claim 1,
wherein a thickness of the cell encapsulation layer is 100 nm to 200 nm.

8. The cell encapsulation layer according to claim 1,
wherein a molecular weight of the chitosan is 15 kDa to 310 kDa.

9. The cell encapsulation layer according to claim 1,
wherein a molecular weight of the hyaluronic acid is 1.2 kDa to 8,000 kDa.

10. A composition for cell encapsulation, comprising the cell encapsulation layer according to any one of claims 1 to 9, and cells.

11. The composition for cell encapsulation according to claim 10,
wherein the chitosan is in direct contact with the cells.

12. The composition for cell encapsulation according to claim 10, comprising at least two cell encapsulation layers.

13. The composition for cell encapsulation according to claim 10,
wherein the cell encapsulation layer includes 1 to 5 chitosan layers and 1 to 5 hyaluronic acid layers.

14. A method of preparing the composition for cell encapsulation according to any one of claims 10 to 13, the method comprising the steps of:
preparing a solution containing (i) a chitosan functionalized with a first moiety group and (ii) a hyaluronic acid functionalized with a second moiety group;
adding an enzyme to the solution to crosslink the polymers (i) and (ii); and
adding cells to the solution before, during or after addition of the enzyme.

15. The method according to claim 14,
wherein the first moiety group and the second moiety group each independently contain phenol, catechol, or quinone.

16. The method according to claim 14,
wherein the first moiety group and the second moiety group contain the phenol.

17. The method according to claim 14,
wherein the cross-linking includes an aryloxy coupling.

18. The method according to claim 14,
wherein the enzyme includes tyrosinase.

19. The method according to claim 14,
wherein the enzyme includes tyrosinase derived from Streptomyces avermitilis (Streptomyces avermitilis-derived tyrosinase) .

20. The method according to claim 14,
wherein the polymer (i) in the solution contains 0.01% to 5% of a chitosan modified with at least one aryl group.

21. The method according to claim 14,
wherein the polymer (ii) in the solution contains 0.01% to 5% of a hyaluronic acid modified with at least one aryl group.

22. Cells that are encapsulated with the cell encapsulation layer according to any one of claims 1 to 9.
